Europäisches Patentamt

(19) European Patent Office

Office européen des brevets

(11) Publication number: **0 293 208 B1**

(12) # EUROPEAN PATENT SPECIFICATION

(45) Date of publication of patent specification :
24.07.91 Bulletin 91/30

(51) Int. Cl.⁵ : **A61L 15/00**

(21) Application number : **88304801.9**

(22) Date of filing : **26.05.88**

(54) Absorptive article.

The file contains technical information
submitted after the application was filed and
not included in this specification

(30) Priority : 27.05.87 JP 132069/87
01.12.87 JP 303478/87
01.12.87 JP 183012/87 U

(43) Date of publication of application :
30.11.88 Bulletin 88/48

(45) Publication of the grant of the patent :
24.07.91 Bulletin 91/30

(84) Designated Contracting States :
AT BE CH DE ES FR GB IT LI NL SE

(56) References cited :
EP-A- 0 210 570
FR-A- 2 203 827
FR-A- 2 380 783
GB-A- 1 570 485
US-A- 3 512 530

(73) Proprietor : **LION CORPORATION**
**3-7, Honjo 1-chome**
**Sumida-ku Tokyo (JP)**

(72) Inventor : **Uchida, Akio**
**c/o Corpo. Shimizusaki 107 Tomuro 97**
**Atsugi-shi Kanagawa (JP)**
Inventor : **Ishihara, Hisako c/o Berupia Isehara**
**1-201**
**19-32, Sakuradai 2-chome**
**Isehara-shi Kanagawa (JP)**
Inventor : **Shinkai, Shigenori**
**c/o Olive Heights 202 30-20, Isehara 3-chome**
**Isehara-shi Kanagawa (JP)**
Inventor : **Kashiwaka, Toshinobu**
**4-2-701, Masago 4-chome**
**Chiba-shi Chiba (JP)**

(74) Representative : **Allam, Peter Clerk et al**
**LLOYD WISE, TREGEAR & CO. Norman House**
**105-109 Strand**
**London WC2R 0AE (GB)**

## Description

The present invention relates to a fluid-absorptive article such as a catamenial napkin or disposable diaper.

A fluid-absorptive article such as catamenial napkin or disposable diaper commercially available today generally comprises a fluid-absorptive material of cotton-like pulp, absorbent paper, hygroscopic polymer or the like, a leakproof material of polyethylene film, waterproof paper or the like adjacent a portion of the outer surface of the fluid-absorptive material, and a fluid-permeable outer surface material.

However, conventional absorptive articles of this kind formed from cotton-like pulp or paper generally do not have the strength and shape-retainability which would be desired, and there is also the problem that the absorptive material tends to be broken or kinked, to form lumps, by the movement of the body as the absorptive article is being worn. Moreover, these problems are most conspicuous after a body fluid such as catamenial blood or urine has been absorbed, which accounts for the principal cause for the leakage of catamenial blood or urine.

With these problems in mind, it has been proposed to use polyurethane foam as the fluid-absorptive material (see Japanese Patent Laid Open, Gazette No. 57-150958 and Japanese Patent Laid Open, Gazette No. 60-42416). Although such use of polyurethane foam has some advantages over cotton-like pulp, it has inferior absorptive properties, such as a poorer rate of absorption of, and a lower absorption capacity for, body fluids. Moreover, it is liable to generate noxious gasses when the used article is incinerated. Accordingly, the use of polyurethane foam cannot be judged to be satisfactory when it is evaluated from the overall viewpoint.

US-A-3954493 and FR-A-2203827 show that it is known in the art to use compressed cellulose-based sponge sheet material in the manufacture of absorptive articles for absorbing fluids. There is a similar disclosure in US-A-3512530.

In accordance with the present invention there is provided an improved absorptive article based on compressed cellulose-based sponge sheet material.

More particularly, the absorptive article in accordance with the invention comprises fluid absorptive material formed by laminating a plurality of cellulose-based compressed sheets which have been provided with slits in order to enhance the flexibility and increase the fluid-absorbing area of the fluid absorptive material.

As the Tests and Example below demonstrate, by using compressed cellulose-based sponge sheet material in the articles of the present invention it is possible to obtain an absorptive article of improved strength and shape retainability when wetted, particularly as compared with the use of cotton-like pulp or absorbent paper as the fluid-absorptive material. Absorptive articles made in accordance with this invention can thus be more comfortable to wear, since they have a reduced tendency to lumpiness when wet. Moreover, and unlike polyurethane foam, cellulose-based sponge material does not give rise to noxious gases when incinerated.

It is preferred that the compressed cellulose-based sponge sheet should have a density in the range of 0.1 to 0.8 g/cm$^3$, more preferably 0.3 to 0.6 g/cm$^3$, and a percentage of voids in the range of 40% to 90%, more preferably from 50% to 80%.

Further, by forming fluid-absorptive material by laminating together a plurality of compressed cellulose-based sponge sheet, the flexibility of the material can be increased as compared with the use of a single thick sheet, so that in the case of articles such as catamenial napkins and disposable diapers a more satisfactory sensation of fitted wear as well as an improved absorptivity of body fluids can be obtained.

Finally, by the provision of slits, in various patterns, in the cellulose-based sponge sheets, the comfort of fitted wear can be improved still further as a result of the flexibility of the fluid-absorptive material being enhanced, and the rate of fluid absorption increased by increasing the fluid-absorbing area.

Moreover, by including a hygroscopically swelling polymer in the fluid-absorptive material it is possible to markedly increase the absorption capacity for body fluids, as well as help to prevent exudation of the body fluid to the outside.

Embodiments of the present absorptive article will now be described in detail with reference to the accompanying drawings, in which :

Fig. 1 is a sectional view of one embodiment of an absorptive article in accordance with the present invention ;

Fig. 2 is a sectional view of another embodiment of absorptive article of the present invention ;

Fig. 3 is a plan view of a fluid-absorptive material of this invention and showing the slits ; and

Figs. 4A to 4Q are plan views showing various alternative slit patterns for the fluid-absorptive material.

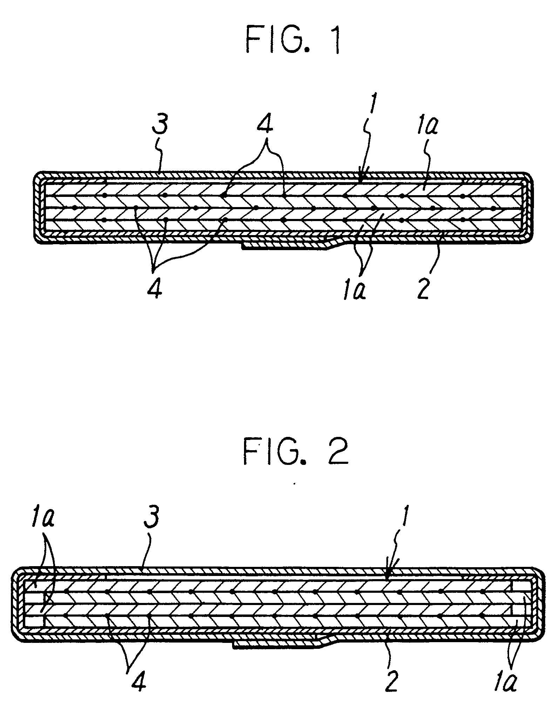

The absorptive article shown in Fig. 1 comprises a fluid-absorptive material 1 for absorbing and holding a body fluid such as catamenial blood or urine, a leakproof material 2 consisting of a fluid-impermeable material such as polyethylene film or waterproof paper, and an outer surface material 3 consisting of a fluid-permeable material such as woven fabric.

The fluid-absorptive material 1 is formed by laminating a plurality of compressed cellulose-based sponge sheets 1a (here illustrated as four sheets) obtained by compressing cellulose-based sponge. A hygroscopically swelling polymer 4 is included in the fluid-absorptive material 1.

The cellulose-based sponge is a sponge of a material containing a cellulose skeleton. Examples of such sponges include, in addition to sponges consisting of cellulose itself, sponges consisting of a cellulose derivative such as viscose, a cellulose ether and a cellulose ester, and sponges consisting of mixtures of those materials just mentioned. By compressing such cellulose sponges with a roller, there is formed a compressed cellulose-based sponge sheet 1a having a network structure which contains air bubbles. It is preferred that the compressed material should have a density of 0.1 to 0.8 g/cm³, more preferably 0.3 to 0.6 g/cm³, and a percent of voids of from 40%, more preferably of 50% to 80%. The absorber 1 here illustrated is formed by laminating a plurality of the sheets 1a, which preferably have a thickness of about 0.05-2.0 mm.

An example of one suitable method for obtaining the cellulose-based sponge is as follows :

(1) Cellulose is converted to alkali cellulose with an alkali, then cellulose xanthate is obtained by subjecting the product to xanthation using carbon disulfide, and viscose is prepared by further adding sodium hydroxide solution. Glauber's salt, reinforcing fiber, and others are added to the viscose which is obtained by the dissolving cellulose, the mixture is extruded thinly by the use of a device such as a T-die, and the product is solidified by heating. The product is washed with water, treated with diluted sulfuric acid solution, washed again with water, neutralized with sodium carbonate and washed with water again, and dried to obtain cellulose-based sponge.

(2) Cellulose-based sponge of predetermined thickness is obtained by slicing, with a slicer, a thick sheet-form or block-form cellulose-based sponge prepared as described in step (1). It is desirable to slice the sponge in a moisture-bearing state and dry it after slicing.

Prior to compressing the cellulose-based sponge obtained in this way, it typically has a density in the range of 0.01 to 0.2 g/cm³ and a compressive deformation factor in the range of 10% to 60% under a load of 45 g/cm³.

As compared with a fluid-absorptive material 1 which is formed by a single thick sheet of compressed cellulose-based sponge, the flexibility and hence the wear comfort of the article as a whole are improved in the fluid-absorptive material of this invention which is made by using a plurality of thinner compressed cellulose-based sponge sheets, as shown in Figures 1 and 2, ie so as to form a laminate. In addition, the absorptivity of body fluid is greater because each sheet 1a is relatively thin, and penetration of the absorbed body fluid to the various layers is facilitated, so that the rate of absorption of the fluid-absorptive material as a whole is high.

Further, by the use of a plurality of the compressed cellulose-based sponge sheets 1a in accordance with this invention, the absorptive article can not only be formed compactly but it also has improved absorptivity characteristics, such as the rate of absorption and the volume of absorption, of body fluids. This is because when the article makes contact with body fluid, the fluid-absorptive material 1 absorbs the body fluid quickly and with a large capacity while its own volume increases substantially, generally from about 2 to 20 times, and usually from 5 to 15 times its volume at the time of compression.

As the hygroscopically swelling polymer 4 which is incorporated in the compressed cellulose-based sponge in the preferred embodiments of this invention, it is desirable to employ a polymer which can absorb water to more than 10 times its own weight. Such polymers are known and include, for example, the hydrolysis product of acrylonitrile grafted starch, crosslinked body of polyacrylate, polyvinyl alcohol, block copolymer of acrylic acid (acrylate), crosslinked body of carboxymethyl cellulose, and denatured polyvinyl alcohol. The hygroscopically swelling polymer 4 may be used in various forms such as that of granules, powders, and fibers, there being nothing critical about the form. Further, the hygroscopically swelling polymer 4 may be combined with the fluid-absorptive material 1 in numerous ways, including, for example, by dispersing it in the sponge, by interposing it between the sheets, and by impregnating the sponge with a monomer of the hygroscopically swelling polymer and then allowing it to be incorporated in the sponge subsequent to the polymerization and crosslinking reactions that take place in this state. The desirable content of the hygroscopically swelling polymer in the absorber is usually in the range of 5% to 70% by weight.

In addition, another method for enhancing the absorption characteristic of the fluid-absorptive sponge material 1 is by introducing a substitution group such as carboxyl group into the sponge in a post-forming process or in the manufacturing process.

In laminating a plurality of compressed cellulose-based sponge sheets, the wear comfort of the article can be enhanced further by alternately staggering these sheets slightly in the widthwise direction, as shown in Fig. 2, although they may be laminated by precisely overlapping them in the vertical direction. In addition, as another method of achieving lamination, a compressed cellulose-based sponge with a predetermined width can be obtained by folding back, toward the centre, the ends of compressed cellulose-based sponge sheets of greater width.

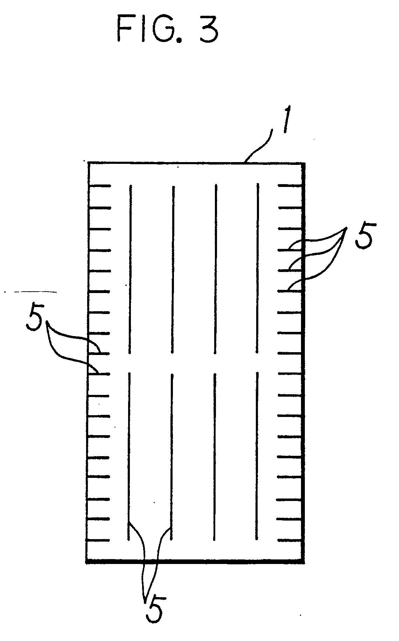

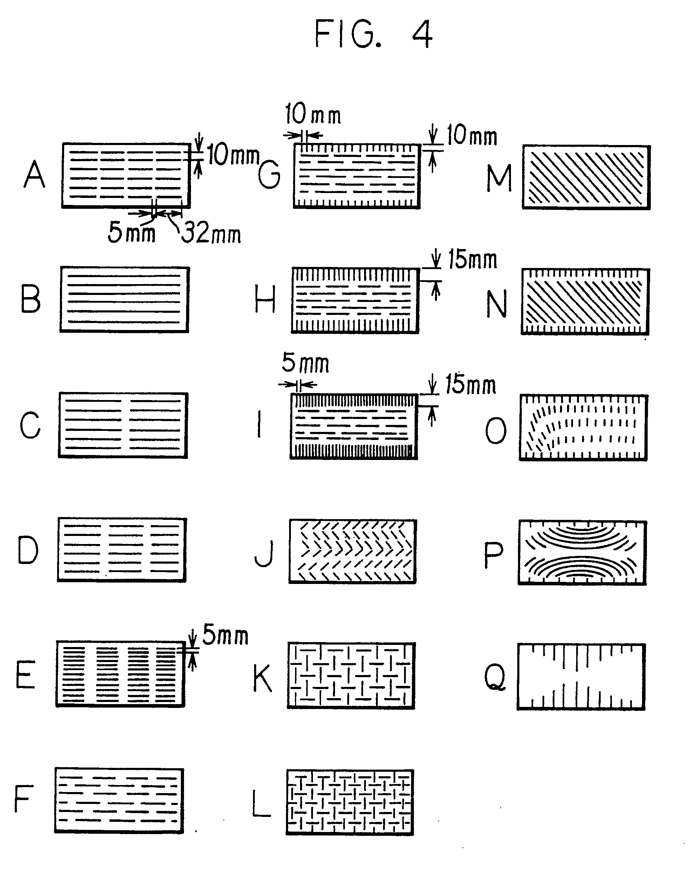

In the compressed cellulose-based sponge sheets from which the fluid-absorptive material is made, a plurality of slits with appropriate spacing is provided, as illustrated by the plan view of Fig. 3. The slits 5 further increase the flexibility of the material 1 which in turn enhances further the sensation of a better fit, as well as increasing the rate of absorption by enlarging the area for absorption of body fluids. That is, body fluid is absorbed not only through the side faces of the slits 5, but also through the rear surface of the fluid-absorptive material 1 which it reaches via the slits 5, so that the rate of absorption is enhanced as compared with the case where no slits are provided. The length, form, direction, and so forth of the slits can be widely varied, and Figs. 4A to 4Q illustrate a number of different slit patterns which can be employed.

The invention is further illustrated by the following Test and Example.

Test 1

Viscose sponge (bulk density of 0.045 g/cm$^3$) of thickness 10 mm was compressed with a roller to form a number of sheets with densities in the range of 0.3 to 0.6 g/cm$^3$. The resulting sheets were then used as fluid-absorptive material in catamenial napkins which were formed by wrapping the sides, rear surface, and a part of the front surface of the sheet with a leakproof material, and then covering the entire surface with an outer fluid-permeable surface material consisting of nonwoven fabric.

The ratio of absorption, capacity for absorption, and wet strength of the resulting catamenial napkins were measured and compared with the corresponding values for conventional catamenial napkins that had an absorber consisting of cotton-like pulp. The results are shown in Table 1.

The rate of absorption, capacity for absorption and wet strength of the samples were measured as follows:

Rate of Absorption

The time required for a drop of physiological saline solution to be absorbed by a sample.

Absorption Capacity

A sample is soaked for 5 minutes under a load of 8 g/cm$^2$ in a vessel containing a physiological saline solution at 37°C, taken out from the vessel and after draining for 10 minutes its weight is measured, to find the ratio of "the weight after the procedure described hereinbefore" to "the weight before soaking".

Wet Strength

Fluid-absorptive material removed from a sample which underwent the absorption capacity test, is cut into a piece having a width of 25 mm, which is subjected to rupture strength measurement under a tensiling speed of 200 mm/min.

### TABLE 1

| Sample No.: | | Material of Fluid-Absorp-tive Material | Density After Compre-ssion $(g/cm^3)$ | Rate of Absorp-tion (sec) | Absorp-tion Capacity $(g/g)$ | Wet Strength $(g)$ |
|---|---|---|---|---|---|---|
| *1 | 1 | Viscose Sponge | 0.30 | Less than 1 sec | 20 | 1000 |
| | 2 | ditto | 0.40 | ditto | 20 | 1000 |
| | 3 | ditto | 0.45 | ditto | 18 | 1000 |
| | 4 | ditto | 0.50 | ditto | 16 | 1050 |
| | 5 | ditto | 0.60 | ditto | 15 | 1100 |
| *2 | a | Cotton-like Pulp | 0.30 | ditto | 7 | 28 |
| | b | ditto | 0.40 | ditto | 6 | 30 |
| | c | ditto | 0.45 | ditto | 6 | 40 |
| | d | ditto | 0.50 | ditto | 5 | 45 |
| | e | Polyure-thane Sponge *3 | 0.50 | More than 60 sec. | 2 | 4000 |

*1 Samples using compressed cellulose-based sponge as required by present invention

*2 Comparative samples

*3 Recovery after compression was too large such that it was not possible to prepare a compressed sheet

Test 2

After dispersing a hygroscopically swelling polymer in the density range of 0.003 to 0.015 $g/cm^2$ in viscose sponge (with pore diameter of 1-3 mm) having a bulk density of 0.045 $g/cm^3$ and a thickness of 10 mm, a fluid-absorptive material was formed by compressing the sponge with a roller to a density of 0.45 $g/cm^3$. Various samples of catamenial napkins were then obtained by wrapping the side faces, rear surface, and a part of the front surface of the compressed sponge material with a leakproof material and then further covering its entire surface with a surface material consisting of woven fabric.

The rate of absorption and the capacity of absorption of these catamenial napkins were measured by the same testing methods as for Test 1. The results are shown in Table 2.

TABLE 2

| Sample No. | Dispersed Amount of Highly Absorbent Polymer $(g/cm^2)$ | Rate of Absorption (sec) | Absorption Capacity $(g/g)$ |
|---|---|---|---|
| 6 | 0.003 | Less than 1 sec. | 25 |
| 7 | 0.007 | ditto | ·28 |
| 8 | 0.015 | ditto | 33 |

From the results of Test 1 and 2 it will be seen that the volume of absorption and the rate of absorption can be improved remarkably by forming the fluid-absorptive material with compressed bodies of cellulose-based sponge sheet, and moreover, that the volume of absorption can further be improved by incorporating a hygroscopically swelling polymer into the fluid-absorptive material.

Test 3

Viscose sponge (bulk density 0.045 g/cm³) of thickness 10 mm was sliced with a slicer and sheets of various thickness and density of 0.4 g/cm³ were formed by compressing the slices with a roller. A number of different fluid-absorptive materials were formed by laminating a plurality of the resultant sheets. Next, a number of catamenial napkins were formed by wrapping the side faces, rear surface, and a part of the front surface of each of the laminated sheet materials and then covering the entire surface with a surface material consisting of non-woven fabric.

The capacity for absorption, the rate of absorption, and the wear-feeling of the resulting catamenial napkin samples were measured and compared with a similar catamenial napkin that had an absorber consisting of a single sheet of cellulose-based sponge. The results are shown in Table 3.

The measurement of the capacity for absorption, the rate of absorption, and the wear-comfort were made by the following methods :

Capacity for Absorption

The method of Test 1 was used.

Rate of Fluid Absorption

An iron plate having a hole surrounded by a cylinder at its middle part is placed over a sample laid horizontally. The rate of fluid absorption is defined as the time required for 10 mℓ of a simulated catamenial blood (concentration of electrolytes 0.89% and surface tension of 42 dyne/cm) poured at a stroke through the cylinder over the sample, under a state of applied pressure of 8 g/cm², to be absorbed through the surface.

Wear Comfort

The comfort in use of the sample surface was determined as the overall average of the results reported by 5 female subjects. The ratings used were as given below :

```
Very comfortable   ...............   5 points
Comfortable   ...................   4 points
Average   .......................   3 points
Uncomfortable   .................   2 points
Very uncomfortable   ...........   1 point
```

The evaluation point was taken as the mean of the 5 subjects.

## TABLE 3

| Sample | Thickness of the lamination (mm) | Absorption Capacity (g/g) | Rate of Fluid Absorption (sec) | Comfort Feeling |
|---|---|---|---|---|
| Lamination of 2 sheets of thickness 0.50 mm | 1.0 | 20 | 20 | 3.0 |
| Lamination of 3 sheets of thickness 0.33 mm | 1.0 | 20 | 19 | 4.0 |
| Lamination of 4 sheets of thickness 0.25 mm | 1.1 | 20 | 19 | 4.2 |
| Lamination of 5 sheets of thickness 0.20 mm | 1.2 | 20 | 18 | 4.2 |
| Lamination of 7 sheets of thickness 0.14 mm | 1.2 | 20 | 17 | 4.5 |
| Lamination of 10 sheets of thickness 0.10 mm | 1.4 | 20 | 17 | 4.5 |
| Single sheet of thickness 1 mm | 1.0 | 20 | 28 | 2.0 |

From the results of Test 3 it can be understood that the rate of absorption of the body fluid and the feeling of wear of the sponge can be improved when the fluid-absorptive material is formed as a laminated body of compressed cellulose-based sponge sheets.

<u>Example</u>

Viscose sponge (bulk density 0.045 g/cm³) of thickness 3.6 mm was sliced into 3 pieces of thickness 1.2 mm, and after compressing each sheet with a roller, 3 sheets were laminated and slits were provided according to a prescribed pattern. The side faces, the rear surface, and a part of the front surface of each laminated structure were wrapped with a leakproof material, and the entire surface was covered with a surface material consisting of non-woven fabric, to obtain a number of catamenial napkins having fluid-absorptive materials with different slit patterns. The slit patterns employed are as shown in Figs. 4A to 4Q. The wear comfort of the resulting products were then evaluated as described in Test 3, and results are shown in Table 4.

TABLE 4

| Slit Pattern | Comfort Rating | Note | Slit Pattern | Comfort Rating | Note |
|---|---|---|---|---|---|
| Fig. 4A | 4.0 | | Fig. 4J | 3.0 | |
| Fig. 4B | 4.5 | *1 | Fig. 4K | 3.0 | |
| Fig. 4C | 3.0 | | Fig. 4L | 3.0 | |
| Fig. 4D | 4.0 | | Fig. 4M | 4.5 | *1 |
| Fig. 4E | 4.0 | | Fig. 4N | 4.5 | *1 |
| Fig. 4F | 4.0 | | Fig. 4O | 3.0 | |
| Fig. 4G | 4.5 | *2 | Fig. 4P | 4.0 | |
| Fig. 4H | 4.5 | *2 | Fig. 4Q | 3.0 | |
| Fig. 4I | 4.5 | *2 | No Slit | 2.0 | |

*1 The comfort was good, but deformation sometimes developed while being worn

*2 The comfort was reported to be exceptionally good.

From the results of this Example it can seen that wear comfort is improved still further by the provision of the slits, as taught by the present invention.

Although the illustrated embodiments of the present invention employ compressed cellulose-based sponge sheet as the sole material for the fluid-absorptive material, it is within the scope of this invention to use other, conventional, fluid-absorptive materials in conjunction with compressed cellulose-based sponge sheets.

**Claims**

1. An absorptive article for absorbing fluids and comprising fluid absorptive material (1) formed from compressed cellulose-based sponge sheet (1a) obtained by compressing cellulose-based sponge, characterized in that said fluid-absorptive material (1) is formed by laminating a plurality of cellulose-based compressed sheets (1a), and in that slits (5) are provided in said compressed cellulose-based sponge sheets thereby to enhance the flexibility and increase the fluid-absorbing area of said fluid absorbtive material (1).

2. An absorptive article as claimed in Claim 1, wherein the density of the compressed cellulose-based sponge sheet is in the range of 0.1 to 0.8 g/cm³, preferably in the range 0.3 to 0.6 g/cm³.

3. An absorptive article as claimed in Claim 1 or Claim 2, wherein the compressed cellulose-based sponge sheet has a void percentage of from 40%-90%, preferably from 50% to 80%.

4. An absorptive article as claimed in any preceding claim, wherein the compressed cellulose-based sponge sheet contains a hygroscopically swelling polymer (4).

5. An absorptive article as claimed in any preceding claim, wherein the fluid-absorptive material is surrounded by an outer layer (3) of fluid-permeable material.

6. An absorptive article as claimed in Claim 5, comprising also a layer (2) of fluid-impermeable material between a portion of said outer layer of fluid-permeable material and said fluid-absorptive material.

7. An absorptive article as claimed in Claim 5 or Claim 6 in the form of a catamenial napkin.

**Patentansprüche**

1. Absorbierender Artikel zum Absorbieren von Flüssigkeiten sowie bestehend aus flüssigkeitsabsorptionsfähigem, aus komprimierter Schwammschicht (1a) auf Zellulosebasis gebildetem Material (1), das durch Komprimieren von Schwamm auf Zellulosebasis gewonnen wird, dadurch **gekennzeichnet**, daß das flüssigkeitsabsorptionsfähige Material (1) durch Laminieren mehrerer komprimierter Schichten (1a) auf Zellulosebasis gebildet wird und daß Schlitze (5) in den komprimierten Schwammschichten auf Zellulosebasis vorgesehen werden, wodurch die Flexibilität erhöht und der flüssigkeitsabsorbierende Bereich des flüssigkeitsabsorptionsfähigen Materials (1) vergrößert wird.

2. Absorbierender Artikel nach Anspruch 1, dadurch **gekennzeichnet**, daß die Dichte der komprimierten Schwammschicht auf Zellulosebasis im Bereich von 0,1 bis 0,8 g/cm³, vorzugsweise im Bereich von 0,3 bis 0,6 g/cm³ liegt.

3. Absorbierender Artikel nach einem der vorhergehenden Ansprüche 1 oder 2, dadurch **gekennzeichnet**, daß die komprimierte Schwammschicht auf Zellulosebasis einen Hohlraum- oder Porenprozentsatz von 40% bis 90%, vorzugsweise von 50% bis 80% besitzt.

4. Absorbierender Artikel nach einem der vorhergehenden Ansprüche 1 bis 3, dadurch **gekennzeichnet**, daß die komprimierte Schwammschicht auf Zellulosebasis ein hygroskopisch quellendes Polymer (4) enthält.

5. Absorbierender Artikel nach einem der vorhergehenden Ansprüche 1 bis 4, dadurch **gekennzeichnet**, daß das flüssigkeitsarbsorptionsfähige Material von einer äußeren Lage (3) flüssigkeitsdurchlässigen Materials umgeben ist.

6. Absorbierender Artikel nach Anspruch 5, **gekennzeichnet** durch eine weitere Lage (2) flüssigkeitsdurchlässigen Materials zwischen einem Abschnitt der äußeren Lage des flüssigkeitsdurchlässigen Materials und dem flüssigheitsabsorptionsfähigen Material.

7. Absorbierender Artikel nach einem der vorhergehenden Ansprüche 5 oder 6, **gekennzeichnet** durch die Ausbildung in Form einer Monatsbinde.

**Revendications**

1. Article absorbant pour absorber les fluides et comportant une matière absorbante de fluide (1) formée à partir de feuille-éponge à base de cellulose comprimée (1a) obtenue en comprimant de l'éponge à base de cellulose, caractérisée en ce que, ladite matière absorbante de fluide (1) est formée par le laminage d'une série de feuilles à base de cellulose comprimée (1a), et en ce que des fentes (5) sont prévues dans lesdites feuilles-éponge à base de cellulose comprimée, améliorant ainsi la souplesse et augmentant la superficie absorbante de fluide de ladite matière absorbante de fluide (1).

2. Article absorbant selon la revendication 1, dont la densité de la feuille d'éponge à base de cellulose comprimée est dans la fourchette de 0,1 à 0,8 g/cm³, et de préférence dans la fourchette de 0,3 à 0,6 g/cm.

3. Article absorbant selon la revendication 1 ou la revendication 2, dont la feuille d'éponge à base de cellulose comprimée prévoit un pourcentage de vide de 40% à 90% et de préférence de 50% à 80%.

4. Article absorbant selon l'une ou l'autre des revendications précédentes, dont la feuille éponge à base de cellulose comprimée comporte un polymère susceptible de dilatation hygroscopique (4).

5. Article absorbant selon l'une ou l'autre des revendications précédentes, dont la matière absorbante de fluide est entourée d'une couche extérieure (3) de matière perméable au fluide.

6. Article absorbant selon la revendication 5, comportant également une couche (2) de matière imperméable au fluide entre une portion de ladite couche extérieure de matière perméable au fluide et ladite matière absorbante de fluide.

7. Article absorbant selon la revendication 5 ou la revendication 6 sous forme de couche cataméniale.

# FIG. 1

# FIG. 2

FIG. 3

# FIG. 4